# EUROPEAN PATENT SPECIFICATION

(11) **EP 3 076 921 B1**
(45) Date of publication and mention of the grant of the patent: **04.03.2020**
(21) Application number: 13812279.1
(22) Date of filing: 02.12.2013
(51) Int. Cl.: A61K 8/27, A61Q 11/00, A61P 1/02, A61K 8/365

(54) **ORAL CARE ZINC COMPOSITIONS**
ZINKZUSAMMENSETZUNGEN ZUR MUNDPFLEGE
COMPOSITIONS DE SOINS BUCCAUX À BASE DE ZINC

(43) Date of publication of application: 12.10.2016
(73) Proprietor: Colgate-Palmolive Company, New York, NY 10022 (US)
(72) Inventor: YANG, Ying, Monmouth Junction New Jersey 08852 (US); TRIVEDI, Harsh M., Hillsborough New Jersey 08844 (US); XU, Guofeng, Plainsboro New Jersey 08536 (US)
(74) Representative: Wibbelmann, Jobst
(86) International application number: PCT/US2013/072671
(87) International publication number: WO 2015/084314

(56) References cited:
- WO-A1-2011/007551
- WO-A2-2010/138544
- WO-A2-2011/088199
- GB-A- 1 290 627
- GB-A- 2 243 775
- US-A1- 2012 034 280
- BATES D G ET AL: "Chemotherapeutic effect of zinc on Streptococcus mutans and rat dental caries", ARCHIVES OF ORAL BIOLOGY, PERGAMON PRESS, OXFORD, GB, vol. 24, no. 10-11, 1 January 1979 (1979-01-01), pages 799-805, XP026154680, ISSN: 0003-9969, DOI: 10.1016/0003-9969(79)90041-4 [retrieved on 1979-01-01]
- VITKOV L ET AL: "Bacterial internalization in periodontitis", ORAL MICROBIOLOGY AND IMMUNOLOGY, vol. 20, no. 5, October 2005 (2005-10), pages 317-321, XP9194838, ISSN: 0902-0055

## Description

### BACKGROUND

Periodontal diseases are chronic bacterial infections and can affect the gingival tissue, periodontal membrane, cementum and the alveolar bone. Diseases such as gingivitis and periodontitis ate complex, multifactorial and multiple stage disorders involving bacteria and host cell interactions. Gingivitis (the inflammation of the gums or gingiva) can lead to periodontitis (inflammation of the periodontium) if untreated. Bacteria present in plaque provoke an inflammatory and/or immune response which can eventually lead to destruction of bone and gum tissue. Periodontitis can lead to alveolar bone loss and eventual tooth loss if untreated.

Epithelial cells provide a protective barrier function against microbial attack in locations such as the oral cavity. The oral cavity is lined with the oral mucosa mucous membrane, made up of stratified squamous epithelium cells together with underlying connective tissue. The gingiva (gums) are part of this soft tissue lining and surround the teeth. Certain pathogens possess the ability to adhere to and invade periodontal cells and tissues. Colonisation by such pathogens can lead to tissue damage by cytotoxic compounds produced by the pathogen as well as indirect damage from the host's immune response.

Dental hygiene measures such as toothbrushing, use of dental floss and the removal of dental plaque by dental hygienists are used to prevent and treat periodontal diseases. Antibacterial agents such as triclosan are also known to be useful. There is however an ongoing requirement to provide alternative and improved ways of treating and preventing periodontal diseases.

US 2012/034280 A1 discloses oral care compositions comprising a first component comprising at least one Eₕ-raising compound and a second component comprising at least one zinc compound and an anti-microbial agent.
WO 2011/007551 A1 concerns a composition comprising zinc chloride.
GB 2243775 A relates to oral compositions comprising therapeutically active metal ion delivering compounds. Said composition may comprise zinc chloride.
GB 1290627 A relates to mouthwash compositions having activity against calculus and plaque comprising a zinc salt.
WO 2010/138544 A2 relates to an oral care composition comprising a film entrained in a carrier, a zinc-containing compound contained in the film, a polysaccharide thickening agent, and a maleic anhydride copolymer.
WO 2011/088199 A2 relates to an oral care composition comprising zinc oxide entrained in a film.
BATES D.G. et al: "Chemotherapeutic effect of zinc on Streptococcus mutans and rat dental caries", ARCHIVES OF ORAL BIOLOGY, PERGAMON PRESS, OXFORD, GB, vol. 24, no. 10-11, 1 January 2079, pages 799-805 reports the chemotherapeutic effect of zinc on Streptococcus mutans and rat dental caries. The zinc salt investigated is zinc sulfate.
Vitkov L. et al: "Bacterial internalization in periodontitis", Oral Microbiology and Immunology (2005); vol. 20 (5), pages 317-321, is a scientific paper investigating bacterial internalization in periodontitis.

### BRIEF SUMMARY

A first aspect of the present invention provides a composition comprising a zinc ion source for use reducing the occurrence or severity of a periodontal disease by inhibiting the invasion of oral soft tissue epithelial cells by a periodontal pathogen, wherein the zinc ion source comprises zinc citrate in an amount of from 5 µM to 6 mM or zinc oxide in an amount of from 12 µM to 24 mM. The oral soft tissue can be any soft tissue in the oral cavity, such as gingiva, cheeks, or tongue.

Optionally the periodontal pathogen causes, transmits or exacerbates a periodontal disease selected from gingivitis and periodontitis.

Optionally the periodontal pathogen is one or more of *Steptococcus mutans, Streptococcus sobrinus, Streptococcus sanguis, Streptococcus oralis, Porphyromonas gingivalis, Porphyromonas cangingivalis, Actinobacillus actinomycetemcomitans, Tannerella forsythia, Fusobacterium nucleatum and Treponema denticola.* Further optionally the periodontal pathogen is *Porphyromonas gingivalis and*/*or Actinobacillus actinomycetemcomitans.*

Optionally the composition is for use in a mammalian subject. Further optionally the composition is for use in a human subject.

Optionally the composition is an oral care composition. Further optionally the composition is an oral care composition selected from dentifrices, toothpastes, tooth powders, mouth rinses, lozenges, gums, gels, paints, and films.

A further aspect of the invention provides a method for inhibiting the adherence to or invasion of oral soft tissue epithelial cells by a periodontal pathogen, the method comprising applying a composition comprising a zinc ion source to oral soft tissue epithelial tissue. The oral soft tissue can be any soft tissue in the oral cavity, such as gingiva, cheeks, or tongue.

Optionally the periodontal pathogen causes, transmits or exacerbates a periodontal disease selected from gingivitis and periodontitis.

Optionally the periodontal pathogen is one or more of *Streptococcus mutans, Streptococcus sobrinus, Streptococcus sanguis, Streptococcus oralis, Porphyromonas gingivalis, Porphyromonas cangingivalis, Actinobacillus actinomycetemcomitans, Tannerella forsythia, Fusobacterium nucleatum and Treponema denficola.* Further optionally the periodontal pathogen is *Porphyromonas gingivalis* and/or *Actirtobacillus actinomycetemcomitans.*

Optionally the zinc ion source comprises one or more zinc salts selected from zinc chloride, zinc acetate, zinc gluconate, zinc sulphate, zinc fluoride, zinc citrate, zinc lactate, zinc oxide, zinc monoglycerolate, zinc tartrate, zinc pyrophosphate, zinc phosphate, zinc maleate, zinc malate, zinc carbonate, zinc ascorbate, zinc lysine hydrochloride and zinc chloride hydroxide monohydrate (TBZC). Further optionally the zinc ion source comprises one or more zinc salts selected from zinc citrate and zinc oxide. Optionally the zinc ion source comprises a combination of two or more zinc salts. Optionally the zinc ion source comprises any combination of two or more zinc salts selected from zinc chloride, zinc acetate, zinc gluconate, zinc sulphate, zinc fluoride, zinc citrate, zinc lactate, zinc oxide, zinc monoglycerolate, zinc tartrate, zinc pyrophosphate, zinc phosphate, zinc maleate, zinc malate, zinc carbonate, zinc ascorbate, zinc lysine hydrochloride and zinc chloride hydroxide monohydrate (TBZC). Further optionally, the zinc ion source comprises a combination of zinc oxide and zinc citrate.

Optionally the composition comprises between I and 20,000 ppm zinc. Further optionally the composition comprises between 1 and 10,000 ppm, between 1 and 5,000 ppm zinc, between 1 and 2,000 ppm zinc, between 1 and 1,000 ppm zinc, between 1 and 500 ppm zinc, between 1 and 200 ppm zinc, between and 100 ppm zinc, between 1 and 50 ppm zinc, between 1 and 25 ppm zinc, between 1 and 10 ppm, or between 3 and 9 ppm zinc. Further optionally the composition comprises between 4 and 8 ppm zinc. Optionally the composition comprises from 0.0001 to 2.0 weight % zinc. Further optionally the composition comprises from 0.0001 to 1.0 weight % zinc, from 0.0001 to 0.5 weight % zinc, from 0.0001 to 0.2 weight % zinc, from 0.0001 to 0.1 weight % zinc, or from 0.0001 to 0.05 weight % zinc.

Optionally the composition can be applied to oral soft tissue epithelial cells of a mammal. Further optionally the composition can be applied to gingival epithelial tissue of a human.

Optionally the composition can be applied at least once per day.

### DETAILED DESCRIPTION

The following description of the preferred embodiment(s) is merely exemplary in nature and is in no way intended to limit the invention, its application, or uses. The scope of the invention is defined by the appended claims.

As used throughout, ranges are used as shorthand for describing each and every value that is within the range. Any value within the range can be selected as the terminus of the range.

Unless otherwise specified, all percentages and amounts expressed herein and elsewhere in the specification should be understood to refer to percentages by weight. The amounts given are based on the active weight of the material.

It has been surprisingly found that zinc ions can enhance the epithelial barrier function of oral soft tissues epithelial cells, such as gingival epithelial cells, and thus protect oral tissues from periodontal pathogen infection. By inhibiting pathogenic organisms from adhering to or invading the epithelial cells in the oral cavity, oral care benefits can be obtained. These benefits can include for example a reduction in the occurrence and/or severity of periodontal diseases such as gingivitis and periodontitis.

Gingivitis is characterised by classic signs of inflammation. Symptoms of gingivitis may include swollen gums, bright red or purple gums, gums that are tender or painful to the touch, bleeding gums and bad breath (halitosis). Gingivitis can be measured using a gingivitis index. For example, Löe and Silness developed an index in 1963 for the assessment of the gingival condition for recording qualitative changes in the gingiva. The marginal and interproximal tissues are scored separately from 0 to 3 wherein:
0= Normal gingiva;
1= Mild inflammation - slight change in colour and slight oedema but no bleeding on probing;
2= Moderate inflammation - redness, oedema and glazing, bleeding on probing;
3= Severe inflammation - marked redness and oedema, ulceration with tendency to spontaneous bleeding
The bleeding is assessed by probing gently along the wall of soft tissue of the gingival sulcus. The scores of the four areas are averaged to give the Gingival Index (GI) for the tooth. The GI of an individual can be obtained by averaging the values for all teeth. An average score from 0.1.1.0 = mild inflammation; 1.1-2.0 = moderate inflammation and from 2.1-3.0 signifies severe inflammation.

Gingivitis may progress to periodontitis in which the periodontium is inflamed. Inflammation of the periodontium can result in the gum tissues separating from the tooth and a periodontal pocket forming. Pathogenic organisms can then colonize the periodontal pocket, causing further inflammation in the gum tissues and alveolar bone loss. Symptoms of periodontitis may include redness or bleeding of gums, swollen gums, halitosis, gingival recession, deep pockets between the teeth and gums and loose teeth. Periodontitis can be diagnosed by probing the gum tissues (gingiva) around the teeth and by examining X-ray films. The severity of periodontitis refers to the amount of periodontal ligament fibre that has been lost. A scale of clinical attachment loss can be used to assess periodontitis in which 1 - 2 mm attachment loss is classified as mild periodontitis, 3 - 4 mm of attachment loss is classified as moderate periodontitis and 5 or more mm of attachment loss is classified as severe periodontitis.

The compositions of the present invention are used to inhibit pathogenic organisms from adhering to or invading epithelial cells. Thus the barrier function of gingival epithelial cells is enhanced. Invasion affords pathogenic organisms with protection from a host's immune system, and is an important virulence factor. By invading epithelial cells, pathogens can successfully colonize a host, obtaining nutrients from the host and competing with the microbiota. The colonization of epithelial cells by pathogenic organisms can result in tissue injury and an inflammatory response. By blocking adherence or invasion, the inflammation induced by the pathogenic organisms and consequent damage is alleviated. Alleviation of inflammation refers to reduction of the level of gingival inflammation stimulated by pathogen infection or invasion.

Inflammation can be measured using cell-based assays. For example, the presence of PGE₂ can be used as a measure for the severity of gingival inflammation. In a cell-based assay, ELISA can be used to analyse PGE₂ levels in cells (e.g. KB cells or mouse macrophage cell line RAW264.7) which have been treated with the pathogenic organism and an anti-inflammatory composition of interest. In one embodiment the composition may reduce the inflammation level by at least 5 %, 10 %, 15 %, 20 %, 30 %, 40 %, 50 %, 60 %, 70 %, 80 %, 100%, 2-fold, 5-fold, 10-fold or 20-fold. In one embodiment the composition reduces the inflammation level of gingival epithelial cells by 5% to 20-fold, for example 5 % to 5-fold or 5 % to 2-fold.

The compositions can also be used to inhibit the invasion of oral soft tissue cells, such as gingival cells, by pathogenic organisms, or the adherence to oral care soft tissues, such as gingival cells, by pathogenic organisms. Inhibiting invasion refers to decreasing the rate of pathogen invasion into cells when contacted with the composition. In one embodiment, the composition can reduce the rate of pathogen invasion into gingival epithelial cells by at least 5 %, 10 %, 15 %, 20 %, 30 %, 40 %, 50 %, 60 %, 70 %, 80 %, 100%, 2-fold, 5-fold, 10-fold or 20-fold. In one embodiment the composition reduces the rate of pathogen invasion into gingival epithelial cells by 5 % to 20 fold, for example 5 % to 5-fold, 5 % to 2-fold, 5 % to 80 % or 5 % to 70%. Inhibiting adherence refers to decreasing the adherence of pathogen to cells when contacted with the composition. In one embodiment, the composition can decrease the adherence of pathogen to gingival epithelial cells by at least 5 %, 10 %, 15 %, 20 %, 30 %, 40 %, 50 %, 60 % or 70 %, 80 %, 2-fold, 5-fold, 10-fold or 20-fold. In one embodiment the composition reduces the adherence of pathogen to gingival epithelial cells by 5 to 80 %, for example 5 to 70 %.

Periodontal pathogens are organisms capable of causing, transmitting, exacerbating or contributing to periodontal diseases including gingivitis and periodontitis. A periodontal pathogen may be a bacterium. The periodontal pathogen may be Gram-negative or Gram-positive. The periodontal pathogen may be anaerobic or aerobic. In one embodiment the periodontal pathogen is a Gram-negative anaerobic bacterium. In one embodiment, the periodontal pathogen is one or more of *Streptococcus mutans, Streptococcus sobrinus, Streptococcus sanguis, Streptococcus oralis, Porphyromonas gingivalis, Porphyromonas cangingivalis, Actinobacillus actinomycetemcomitans, Tannerella forsythia, Fusobacterium nucleatum and Treponema denticola. P. gingivalis, Tannerella forsythia* and *Treponema denticola* are known together as the "red complex" of pathogens and can operate together within subgingival plaque. Pathogenic organisms such as *Streptococcus mutans, Streptococcus sobrinus, Streptococcus sanguis, Streptococcus oralis, Porphyromonas gingivalis, Porphyromonas cangingivalis, Actinobacillus actinomycetemcomitans, Tannerella forsythia, Fusobacterium nucleatum and Treponema denticola* are capable of colonising the oral cavity and releasing cytotoxic products such as ammonia, organic acids and volatile sulphur compounds which can damage and destroy the tissues of the oral cavity. Although the epithelium has built-in defence systems, pathogenic bacteria can modulate the mucosal epithelial barrier in order to initiate and/or progress periodontal disease. For example, a pathogenic bacterium can target specific host receptors, modulate signalling events in the host and deregulate the host cytokine network in order adhere and invade epithelial cells. It has now been found that zinc can be used to disrupt invasion into and adherence to gingival epithelial cells by pathogenic organisms.

The compositions of the present invention comprise a zinc ion source. The zinc ion source is capable of providing Zn²⁺ ions to the oral cavity, including delivery to a surface in the oral cavity. In one embodiment the zinc ion source is capable of delivering Zn²⁺ ions to the oral mucosa including the gingival epithelia.
The zinc ion source comprises zinc citrate in an amount of from 5 µm to 6 mM or zinc oxide in an amount from 12 µm to 24 mM.

The composition is for use in a mammalian subject. In one embodiment the composition is for use in a human or animal subject. For example, the subject may be selected from humans, sheep, cattle, horses, pigs, poultry, cats and dogs. In one embodiment the composition is for use in a human subject.

In one embodiment the composition is an oral care composition. In one embodiment the oral care composition is selected from dentifrices, toothpastes, tooth powders, mouth rinses, lozenges, gums, gels, paints and films,

The composition may further comprise an oral care active. For example, the composition may comprise an oral care active selected from one or more of a fluoride ion source, an antisensitivity agent, an occlusion agent, a tooth whitening agent, a tooth bleaching agent, an anticalculus agent, an amino acid and mixtures thereof.

Fluoride sources suitable for use in the present invention may include, but are not limited to: ionic fluorides including alkali metal fluorides; amine fluorides such as olaflur (N'-octadecyltrimethylendiamine-N,N,N'-tris(2-ethanol)-dihydrofluoride), indium fluoride, sodium fluoride, potassium fluoride, calcium fluoride, zinc fluoride, zinc ammonium fluoride, lithium fluoride, ammonium fluoride, stannous fluoride, stannous fluorozirconate, sodium monofluorophosphate, potassium monofluorophosphate, laurylamine hydrofluoride, diethylaminoethyloctoylamide hydrofluoride, didecyldimethylammonium fluoride, cetylpyridinium fluoride, dilaurylmorpholinium fluoride, sarcosine stannous fluoride, glycine potassiuym fluoride, glycin hydrofluoride, amine fluoride or combinations thereof; and ionic monofluorophosphates including alkali metal monofluorophosphates such as potassium, sodium and ammonium fluoride and monofluorophosphates and mixtures thereof. In certain embodiments, the oral care composition of the invention may also contain a source of fluoride ions or fluorine-providing ingredient in amounts sufficient to supply about 50 to about 5000 ppm fluoride ion, e.g.. from about 100 to about 1000, from about 200 to about 500, or about 250 ppm fluoride ion. Fluoride ion sources may be added to the compositions of the invention at a level of about 0.001 wt. % to about 10 wt. %, e.g., from about 0.003 wt. % to about 5 wt. %, 0.01 wt. % to about 1 wt., or about 0.05 wt. %. However, it is to be understood that the weights of fluoride salts to provide the appropriate level of fluoride ion will obviously vary based on the weight of the counter ion in the salt, and one of skill in the art may readily determine such amounts. A preferred fluoride salt may be sodium fluoride.

The composition may comprise at least one flavorant, useful for example to enhance taste of the composition. Any orally acceptable natural or synthetic flavorant can be used, including without limitation vanillin, sage, marjoram, parsley oil, spearmint oil, cinnamon oil, oil of wintergreen (methylsalicylate), peppermint oil, clove oil, bay oil, anise oil, eucalyptus oil, citrus oils, fruit oils and essences including those derived from lemon, orange, lime, grapefruit, apricot, banana, grape, apple, strawberry, cherry, pineapple, *etc,* bean- and nut-derived flavors such as coffee, cocoa, cola, peanut, almond.. *etc.,* adsorbed and encapsulated flavorants and the like. Also encompassed within flavorants herein are ingredients that provide fragrance and/or other sensory effect in the mouth, including cooling or warming effects. Such ingredients illustratively include menthol, menthyl acetate, menthyl lactate, camphor, eucalyptus oil, eucalyptol, anethole, eugenol, cassia, oxanone, α-irisone, propenyl guaiethol, thymol, linalool, benzaldehyde, cinnamaldehyde, N-ethyl-*p*-menthan-3-carboxamine, N,2,3-trimethyl-2-isopropylbutanamide, 3-(1-menthoxy)-propane-1,2-diol, cinnamaldehyde glycerol acetal (CGA), menthone glycerol acetal (MGA) and the like. One or more flavorants are optionally present in a total amount of from about 0.01 wt % to about 5 wt. %, for example, from about 0.1 wt. % to about 2.5wt. %, by total weight of the composition.

The composition of the present invention optionally incorporates one or more antisensitivtity agents, e.g., potassium salts such as potassium nitrate, potassium bicarbonate, potassium chloride, potassium citrate, and potassium oxalate; capsaicin; eugenol; strontium salts; chloride salts and combinations thereof. Such agents may be added in effective amounts, e.g., from about 1 wt. % to about 20 wt. % by weight based on the total weight of the composition, depending on the agent chosen.

The composition may further comprise an antioxidant Any orally acceptable antioxidant can be used, including butylated hydroxyanisole (BHA), butylated hydroxytoluene (BHT), vitamin A, carotenoids, vitamin E, flavonoids, polyphenols, ascorbic acid, herbal antioxidants, chlorophyll, melatonin, and mixtures thereof.

The composition of the present invention may additionally optionally comprise a tartar control (anticalculus) agent as provided below. Tartar control agents among those useful herein include salts of the specified agents, including alkali metal and ammonium salts. The agents include: phosphates and polyphosphates (for example pyrophosphates), polyaminopropanesulfonic acid (AMPS), polyolefin sulfonates, polyolefin phosphates, diphosphonates such as azacycloalkane-2,2-diphosphonates (e.g., azacycloheptane-2,2-diphosphonic acid), N-methyl azacyclopentane-2,3-diphosphonic acid, ethane-1-hydroxy-1,1-diphosphonic acid (EHDP) and ethane-1-amino-1,1-diphosphonate, phosphonoalkane carboxylic acids and. Useful inorganic phosphate and polyphosphate salts include monobasic, dibasic and tribasic sodium phosphates, sodium tripolyphosphate, tetrapolyphospbate, mono-, di-, tri- and tetrasodium pyrophosphates, sodium trimetaphosphate, sodium hexametaphosphate and mixtures thereof. Other useful tartar control agents include polycarboxylate polymers and polyvinyl methyl ether/maleic anhydride (PVM/MA) copolymers, such as GANTREZ®. Such agents may be added ineffective amounts e.g. from 1 to 20 weight %.

In some embodiments, the composition may comprise one or more amino acid. Amino acids that may be incorporated into the compositions of the present invention include arginine, histidine, lysine, aspartic acid, glutamic acid, serine, threonine, asparagine, glutamine, cysteine, glycine, proline, alanine, valine, isoleucine, leucine, methionine, phenylalanine, tyrosine, and tryptophan. In some embodiments, the composition comprises one or more amino acid selected from L-arginine, cysteine, isoleucine, L-lysine, glutamic acid, serine and mixtures thereof. In some embodiments, the composition comprises L-arginine.

In some embodiments the composition may comprise a polypeptide. For example, the composition may comprise polylysine comprising 25 - 30 lysine residues.

In some embodiments, the composition of the present invention further comprises a nutrient. Suitable nutrients include vitamins, minerals, amino acids, and mixtures thereof. Vitamins include Vitamins C and D, thiamine, riboflavin, calcium pantothenate, niacin, folic acid, nicotinamide, pyridoxine, cyanocobalamin, para-aminobenzoic acid, bioflavonoids, and mixtures thereof. Nutritional supplements include amino acids (such as L-tryptophan, L-lysine, methionine, threonine, levocaroitine and L-carnitine), lipotropics (such as choline, inositol, betaine, and linoleic acid), and mixtures thereof.

The invention also provides a method for inhibiting the adherence to or invasion of gingival epithelial cells by a periodontal pathogen, the method comprising applying a composition comprising a zinc ion source to gingival epithelial tissue. The composition may be applied to the tissue by hand or by using an implement such as a brush, for example by dipping the brush into the oral care composition and then applying it to the gingival epithelial tissue. Alternatively, the composition may be applied in a liquid mouth rinse. In one embodiment, the composition may be applied to gingival epithelial cells between one and four times per day i.e. between one and four times in a 24 hour period. In one embodiment the composition is applied to gingival epithelial tissue once per day.

### Examples:

### Cell preparation:

Human periodontal ligament fibroblasts (HPdLF, Lonza cat#CC-7049) are seeded onto a plate and incubated at 37°C in 5 % CO₂ overnight using SCGM bullet kit (Lonza, #CC-3205) containing Stromal cell basal medium, hFGF-B, insulin, FBS and GA-1000.

### Preparation of bacteria:

Pathogenic bacteria (*Actinobacillus actinomycetemcomitans* (Aa) ATCC cat#29522 or *Porphyromonas gingivatis* (Pg) ATCC est#33277) are incubated overnight at 37 °C (using tryptic soy broth for *Actinobacillus actinomycetemcomitans* and half-strength brain heart infusion (supplemented with yeast extract, hemin and vitK1) for *Porphyromonas gingivalis*)*.* The optical density (OD) at 610 nm is read. The bacteria are centrifuged at 8000 rpm for 10 mins and the pellet resuspended in cell culture media and diluted to OD₆₁₀ of 0.1 to 0.2.

### Cell infection:

The test sample (dissolved in water) is added to the cells and incubated at 37 C in 5 % CO₂ incubator for 30 minutes before the bacteria are added. The cells are infected with bacteria by adding the resuspended bacteria to the cells and further incubating at 37°C in 5 *%* CO₂ incubator for 90 to 120 minutes.

### Determination of bacterial invasion:

The cells are washed five times with PBS to remove non-adherent bacteria. Fresh cell culture medium containing 1x penicillin (100 IU) and streptomycin (100 µg/ml) is added and the cells incubated at 37 °C in 5 % CO₂ incubator for 2 hours to kill extracellular bacteria. The medium is then removed and the cells washed three times with PBS. 1 ml 1% saponin solution in PBS is added to the infected cells for 5 minutes at 37 °C to lyse the cells and release internalized bacteria. Dilutions (serial dilutions in PBS with dilution factor 10) are plated onto a suitable medium using a 100 µl sample and spread with a sterilized stick before incubating in an anaerobic chamber at 37 °C for 2 - 3 days and counting the resultant bacterial colonies.

Results are shown in Tables 1 and 2. Table 1 shows the effect of zinc oxide on *Actinobacillus actinomycetemcomitans* and *Porphyromonas gingivalis.* Table 1 shows that cells treated with zinc oxide have less internalized *Actinobacillus actinomycetemcomitans* and less internalized *Porphyromonas gingivalis.* In this example, zinc oxide reduces internalized *Actinobacillus actinomycetemcomitans* by approximately 15 % and internalized *Porphyromonas gingivalis* by approximately 6 %. Table 2 shows that both zinc oxide and zinc citrate reduce internalized *Actinobacillus actinomycetemcomitans* invasion into human periodontal ligament fibroblasts. Zinc citrate at 8 ppm reduces internalized *Actinobacillus actinomycetemcomitans* by approximately 66 % and zinc oxide at 4 ppm reduces internalized *Actinobacillus actinomycetemcomitans* by approximately 23 %.

**Table 1**

| Sample | Bacterial Count |
|---|---|
| Aa | 11800 |
| Aa + ZnO | 10040 |
| Pg | 11460 |
| Pg + ZnO | 10760 |

**Table 2**

| Sample | Bacterial Count |
|---|---|
| Aa + ZnCitrate 8 ppm | 64 |
| Aa + ZnO 4 ppm | 137 |
| Aa | 178 |

## Claims

1. A composition comprising a zinc ion source for use in reducing the occurrence or severity of a periodontal disease by inhibiting the invasion of oral soft tissue epithelial cells by a periodontal pathogen, wherein the zinc ion source comprises zinc citrate in an amount of from 5 µM to 6 mM or zinc oxide in an amount of from 12 µM to 24 mM.

2. The composition for use according to claim 1 wherein the periodontal disease is gingivitis and periodontitis.

3. The composition for use according to any preceding claim, wherein the oral soft tissue is gingival epithelial cells.

4. The composition for use according to any preceding claim wherein the periodontal pathogen causes, transmits or exacerbates a periodontal disease selected from gingivitis and periodontitis.

5. The composition for use according to any preceding claim wherein the periodontal pathogen is one or more of Streptococcus mutans, Streptococcus sobrinus, Streptococcus sanguis, Streptococcus oralis, Porphyromanas gingivalis, Porphyromanas cangingivalis, Actinobacillus actinomycetemcomitans, Tannerella forsythia, Fusobacterium nuclaetum and Treponema denticola.

6. The composition for use according to any preceding claim wherein the periodontal pathogen is Porphyromonas gingivalis and/or Actinobacillus actinomycetemcomitans.

7. The composition for use according to any preceding claim wherein the zinc ion source comprises zinc citrate in an amount of from 5 µM to 2.4 mM or zinc oxide in an amount of from 12 µM to 24 mM.

8. The composition for use according to any preceding claim wherein the zinc ion source comprises zinc citrate in an amount of from 5 µM to 1.2 mM or zinc oxide in an amount of from 12 µM to 12 mM.

9. The composition for use according to any preceding claim wherein the zinc ion source comprises zinc citrate in an amount of from 5 µM to 600 µM or zinc oxide in an amount of from 12 µM to 6 mM.

10. The composition for use according to any preceding claim wherein the zinc ion source comprises zinc citrate in an amount of from 5 µM to 300 µM or zinc oxide in an amount of from 12 µM to 2.4 mM.

11. The composition for use according to any preceding claim in a mammalian, subject.

12. The composition for use according to any preceding claim in a human subject.

13. The composition for use according to any preceding claim wherein the composition is an oral care composition.

14. The composition for use according to any preceding claim wherein the composition is an oral care composition selected from dentifrices, toothpastes, tooth powders, mouth rinses, lozenges, gums, gels, paints, and films.

15. The composition for use according to claim 14, wherein the composition is in the form of a mouth rinse.

## Patentansprüche

1. Zusammensetzung umfassend eine Zinkionenquelle zur Verwendung bei der Reduzierung des Auftretens oder der Erschwerung einer periodontalen Erkrankung durch Inhibierung der Invasion von oralen Weichgewebe-Epithelzellen durch ein periodontales Pathogen, wobei die Zinkionenquelle Zinkcitrat in einer Menge von 5 µM bis 6 mM oder Zinkoxid in einer Menge von 12 µM bis 24 mM umfasst.

2. Zusammensetzung zur Verwendung gemäß Anspruch 1, wobei die periodontale Erkrankung Gingivitis und Periodontitis ist.

3. Zusammensetzung zur Verwendung nach irgendeinem vorhergehenden Anspruch, wobei das orale Weichgewebe Gingivalepithelzellen sind.

4. Zusammensetzung zur Verwendung nach irgendeinem vorhergehenden Anspruch, wobei das Periodontal-Pathogen eine periodontale Erkrankung verursacht, überträgt oder verstärkt ausgewählt aus Gingivitis und Periodontitis.

5. Zusammensetzung zur Verwendung gemäß irgendeinem vorhergehenden Anspruch, wobei das Periodontal-Pathogen eines oder mehreres von Streptococcus mutans, Streptococcus sobrinus, Streptococcus sanguis, Streptococcus oralis, Porphyromanas gingivalis, Porphyromanas cangingivalis, Actinobacillus actinomycetemcomitans, Tannerella forsythia, Fusobacterium nuclaetum und Treponema denticola ist.

6. Zusammensetzung zur Verwendung nach irgendeinem vorhergehenden Anspruch, wobei das Periodontal-Pathogen Porphyromonas gingivalis und/oder Actinobacillus actinomycetemcomitans ist.

7. Zusammensetzung zur Verwendung nach irgendeinem vorhergehenden Anspruch, wobei die Zinkionenquelle Zinkcitrat in einer Menge von 5 µM bis 2,4 mM oder Zinkoxid in einer Menge von 12 µM bis 24 mM umfasst.

8. Zusammensetzung zur Verwendung nach irgendeinem vorhergehenden Anspruch, wobei die Zinkionenquelle Zinkcitrat in einer Menge von 5 µM bis 1,2 mM oder Zinkoxid in einer Menge von 12 µM bis 12 mM umfasst.

9. Zusammensetzung zur Verwendung nach irgendeinem vorhergehenden Anspruch, wobei die Zinkionenquelle Zinkcitrat in einer Menge von 5 µM bis 600 µM oder Zinkoxid in einer Menge von 12 µM bis 6 mM umfasst.

10. Zusammensetzung zur Verwendung nach irgendeinem vorhergehenden Anspruch, wobei die Zinkionenquelle Zinkcitrat in einer Menge von 5 µM bis 300 µM oder Zinkoxid in einer Menge von 12 µM bis 2,4 mM umfasst.

11. Zusammensetzung zur Verwendung nach irgendeinem vorhergehenden Anspruch in einem Säugetier-Subjekt.

12. Zusammensetzung zur Verwendung nach irgendeinem vorhergehenden Anspruch in einem humanen Subjekt.

13. Zusammensetzung zur Verwendung nach irgendeinem vorhergehenden Anspruch, wobei die Zusammensetzung eine Mundpflegezusammensetzung ist.

14. Zusammensetzung zur Verwendung nach irgendeinem vorhergehenden Anspruch, wobei die Zusammensetzung eine Mundpflegezusammensetzung ist, ausgewählt aus Zahnpflegemittteln, Zahnpasten, Zahnpulvern, Mundspülungen, Lutschtabletten, Gummis, Gelen, Einfärbungen und Filmen.

15. Zusammensetzung zur Verwendung nach Anspruch 14, wobei die Zusammensetzung in Form einer Mundspülung ist.

## Revendications

1. Composition comprenant une source d'ions zinc pour une utilisation dans la réduction de l'apparition ou de la gravité d'une maladie parodontale en inhibant l'invasion des cellules épithéliales des tissus mous buccaux par un pathogène parodontal, dans laquelle la source d'ions zinc comprend du citrate de zinc dans une quantité allant de 5 µM à 6 mM ou de l'oxyde de zinc dans une quantité allant de 12 µM à 24 mM.

2. Composition pour l'utilisation selon la revendication 1, dans laquelle la maladie parodontale est la gingivite et la parodontite.

3. Composition pour l'utilisation selon l'une quelconque des revendications précédentes, dans laquelle le tissu mou buccal est constitué de cellules épithéliales gingivales.

4. Composition pour l'utilisation selon l'une quelconque des revendications précédentes, dans laquelle le pathogène parodontal provoque, transmet ou exacerbe une maladie parodontale choisie parmi la gingivite et la parodontite.

5. Composition pour l'utilisation selon l'une quelconque des revendications précédentes, dans laquelle le pathogène parodontal est l'un ou plusieurs parmi Streptococcus mutans, Streptococcus sobrinus, Streptococcus sanguis, Streptococcus oralis, Porphyromanas gingivalis, Porphyromanas cangingivalis, Actinobacillus actinomycetemcomitans, Tannerella forsythia, Fusobacterium nuclaetum et Treponema denticola.

6. Composition pour l'utilisation selon l'une quelconque des revendications précédentes, dans laquelle le pathogène parodontal est Porphyromonas gingivalis et/ou Actinobacillus actinomycetemcomitans.

7. Composition pour l'utilisation selon l'une quelconque des revendications précédentes, dans laquelle la source d'ions zinc comprend du citrate de zinc dans une quantité allant de 5 µM à 2,4 mM ou de l'oxyde de zinc dans une quantité allant de 12 µM à 24 mM.

8. Composition pour l'utilisation selon l'une quelconque des revendications précédentes, dans laquelle la source d'ions zinc comprend du citrate de zinc dans une quantité allant de 5 µM à 1,2 mM ou de l'oxyde de zinc dans une quantité allant de 12 µM à 12 mM.

9. Composition pour l'utilisation selon l'une quelconque des revendications précédentes, dans laquelle la source d'ions zinc comprend du citrate de zinc dans une quantité allant de 5 µM à 600 µM ou de l'oxyde de zinc dans une quantité allant de 12 µM à 6 mM.

10. Composition pour l'utilisation selon l'une quelconque des revendications précédentes, dans laquelle la source d'ions zinc comprend du citrate de zinc dans une quantité allant de 5 µM à 300 µM ou de l'oxyde de zinc dans une quantité allant de 12 µM à 2,4 mM.

11. Composition pour l'utilisation selon l'une quelconque des revendications précédentes chez un sujet mammifère.

12. Composition pour l'utilisation selon l'une quelconque des revendications précédentes chez un sujet humain.

13. Composition pour l'utilisation selon l'une quelconque des revendications précédentes, dans laquelle la composition est une composition de soin buccal.

14. Composition pour l'utilisation selon l'une quelconque des revendications précédentes, dans laquelle la composition est une composition de soin buccal choisie parmi les dentifrices, les pâtes dentifrices, les poudres dentifrices, les bains de bouche, les pastilles, les gommes, les gels, les colorations et les films.

15. Composition pour l'utilisation selon la revendication 14, dans laquelle la composition est sous la forme d'un bain de bouche.
